# EUROPEAN PATENT APPLICATION

(11) **EP 3 308 853 A1**
(43) Date of publication of application: **18.04.2018**
(21) Application number: 16807463.1
(22) Date of filing: 07.06.2016
(51) Int. Cl.: B01J 20/22, A61L 9/01, A61L 9/16, B01D 53/14, C07C 211/13, C07C 243/28, C07C 279/00

(54) **KETONE-BASED GAS ADSORBENT, GAS ADSORBENT COMPOSITION, AND DEODORANT PROCESSED GOODS**

(30) Priority: 12.06.2015 JP 2015119588; 12.06.2015 JP 2015119682
(71) Applicant: Toagosei Co., Ltd., Minato-ku Tokyo 105-8419 (JP)
(72) Inventor: SUGIURA Koji, Nagoya-shi Aichi 455-0026 (JP)
(74) Representative: Neuefeind, Regina
(86) International application number: PCT/JP2016/066890
(87) International publication number: WO 2016/199756

(57) **Abstract**

The ketone-based gas adsorbent of the present invention is characterized by containing a primary amine compound. The primary amine compound is preferably at least one selected from the group consisting of hydrazide compounds, aminoguanidine compounds, and polyamines.

## Description

The present invention relates to: an adsorbent which adsorbs ketone-based gases; and a gas adsorbent composition and a deodorant processed article, which contain the adsorbent.

There is a growing interest in daily-life odors, and in-room stationary-type and spray-type deodorant products have been proposed for reduction of unpleasant odor or offensive odor. Further, a variety of deodorant products obtained by imparting a deodorizing effect to wall papers, curtains, carpets, mats, sofas, filters, clothings and the like have been commercialized. In these deodorant products, a specific adsorbent is used in accordance with the type of the unpleasant odor or offensive odor. Ketones, a type of unpleasant odor component, are contained in various odors such as cigarette smoke, body odor (e.g., sweat odor and oral odor), pet odor, mold odor, paint odor, and print odor. Particularly, diacetyl, 2,3-pentanedione, 2,3-hexanedione and the like are known as sweat odor components that are unique to middle-aged men, and acetone, acetoacetic acid, *β*-hydroxybutyric acid and the like are ketone bodies known as body odor components, all of which are unpleasant odors.

Conventionally, activated charcoal has been often used as an adsorbent for reduction of such unpleasant odor and offensive odor components; however, since activated charcoal is black in color and exhibits only a physical adsorption property, it is difficult to use activated charcoal as a general-purpose adsorbent in various applications. Since a gas component adsorbs to all kinds of matters, a drawback of physical adsorption is that, in an open space, activated charcoal is saturated in a short time by continuously adsorbing gases other than offensive odor. Moreover, since saturation of the adsorbed amount or an increase in the ambient temperature may induce release of the once-adsorbed gases and thereby make the activated charcoal an offensive odor source, activated charcoal can only be used in replaceable products. Therefore, adsorbents having a chemical adsorption property have been developed.

As an adsorbent which chemically adsorbs ketone-based gases, for example, Patent Document 1 discloses a body odor suppressing agent containing an ascorbic acid fatty acid ester compound. Patent Document 2 discloses a menstrual odor deodorizing composition containing a plant extract. Patent Document 3 discloses an adsorbent which contains a carrier carrying at least two enzymes (complex enzyme) selected from the group consisting of oxidoreductase, transferase, hydrolase, lyase, isomerase and synthetic enzymes. Moreover, Patent Document 4 discloses a deodorant obtained by coating activated charcoal with an ion-exchange resin.

Patent Document 1: Japanese Patent Application Laid-Open (JP-A) No. 2014-55124
Patent Document 2: JP-ANo. 2001-198200
Patent Document 3: JP-ANo. 2005-246374
Patent Document 4: JP-ANo. 2011-254853

However, these adsorbents such as the ascorbic acid fatty acid ester compound disclosed in Patent Document 1 do not have a sufficient ketone-based gas-adsorbing effect and also present a problem in terms of heat resistance when they are used in combination with a thermoplastic resin or the like to produce a resin molded article.

Generally speaking, in order to obtain sufficient adsorption effect, it is necessary to use an adsorbent in a large amount; however, the use of a large amount of an adsorbent in the production of a resin molded article causes coloration or discoloration and reduces the adsorption effect.

An object of the invention is to provide: a ketone-based gas adsorbent and a gas adsorbent composition, which exhibit high chemical adsorption performance for ketone-based gases and have excellent processability and heat resistance; and a deodorant processed article which exhibits excellent chemical adsorption performance for ketone-based gases.

The present inventor intensively studied to discover that a primary amine compound-containing gas adsorbent exhibits excellent adsorption performance, thereby completing the invention.

The invention includes the followings.
1. A ketone-based gas adsorbent containing a primary amine compound.
2. The ketone-based gas adsorbent according to 1., wherein the primary amine compound is at least one selected from the group consisting of hydrazide compounds, aminoguanidine compounds, and polyamines.
3. The ketone-based gas adsorbent according to 2., wherein the aminoguanidine compounds are aminoguanidine sulfate and aminoguanidine hydrochloride.
4. The ketone-based gas adsorbent according to any one of 1. to 3., which is a complex in which the primary amine compound is supported on an inorganic powder.
5. The ketone-based gas adsorbent according to 4., wherein the inorganic powder is at least one selected from silicate compounds, tetravalent metal phosphate compounds, silica gels, and zeolites.
6. The ketone-based gas adsorbent according to 4. or 5., wherein the inorganic powder has a BET specific surface area of not less than 80 m²/g.
7. The ketone-based gas adsorbent according to any one of 4. to 6., wherein the mass ratio of the primary amine compound and the inorganic powder is from 5:95 to 60:40.
8. A gas adsorbent composition containing: the ketone-based gas adsorbent according to any one of 1. to 7.; and at least one gas adsorbent selected from the group consisting of basic gas adsorbents, sulfur-based gas adsorbents, and organic acidic gas adsorbents.
9. A deodorant processed article containing the ketone-based gas adsorbent according to any one of 1. to 7.

The ketone-based gas adsorbent of the invention chemically adsorbs ketone-based gases and has excellent deodorizing effect against ketone-based gas-containing odors. Especially when the primary amine compound is a hydrazide compound, an aminoguanidine compound or a polyamine, the ketone-based gas adsorbent of the invention has particularly excellent deodorizing effect since it readily undergoes Schiff reaction with ketones. Further, the ketone-based gas adsorbent of the invention can be coated on or kneaded into papers, fibers, resin molded articles and the like, and thus has excellent processability. Moreover, for example, in the production of a deodorant processed article composed of a thermoplastic resin molded article containing a ketone-based gas adsorbent by a method including a melt-kneading process or molding process, the ketone-based gas adsorbent of the invention exhibits a deodorizing effect against ketone-based gases and provides excellent heat resistance.

The gas adsorbent composition of the invention has an effect of deodorizing ketone-based gases as well as other gases. By using the ketone-based gas adsorbent or gas adsorbent composition of the invention, deodorant processed articles (deodorant products) that exhibit excellent adsorption performance, such as papers, nonwoven fabrics, fibers and resin molded articles, as well as paints, spray materials and the like that yield these articles, can be provided.

The deodorant processed article of the invention, when used in a ketone-based gas atmosphere, is not only capable of deodorizing odors but also inhibiting generation of a ketone-based gas from itself.

The invention is described in detail below.

The ketone-based gas adsorbent of the invention is characterized by containing a primary amine compound. The ketone-based gas adsorbent of the invention may consist of only a primary amine compound, or may be composed of a primary amine compound and other material. The outer appearance of the ketone-based gas adsorbent of the invention is of a solid and, when the primary amine compound is liquid, the ketone-based gas adsorbent is a complex composed of the primary amine compound and a solid other material.

In the invention, the ketone-based gases to be adsorbed are ketone gases, such as acetone, methyl ethyl ketone, methyl propyl ketone, methyl butyl ketone, diethyl ketone, methyl amyl ketone, diacetyl, acetoin, 2,3-pentanedione, and 2,3-hexanedione.

The primary amine compound is not particularly restricted; however, it is preferably a compound which undergoes Schiff reaction with ketones. For carrying out the invention, from the standpoints of safety, deodorizing effect, economic efficiency and the like, hydrazide compounds, aminoguanidine compounds and polyamines are suitable as the primary amine compound. These compounds may be used singly, or in combination of two or more thereof.

Examples of the hydrazide compounds include monohydrazide compounds having one hydrazide group in each molecule, dihydrazide compounds having two hydrazide groups in each molecule, and polyhydrazide compounds having three or more hydrazide groups in each molecule.

In the invention, these hydrazide compounds may be used singly, or in combination of two or more thereof.

Examples of the monohydrazide compounds include monohydrazide compounds represented by the following Formula (1):

R¹-CO-NHNH₂ (1)

(wherein, R¹ represents a hydrogen atom, an alkyl group, or an aryl group optionally having a substituent).

In Formula (1), the alkyl group represented by R¹ may be an aliphatic hydrocarbon group having from 1 to 20 carbon atoms and is preferably a linear alkyl group having from 1 to 12 carbon atoms, such as a methyl group, an ethyl group, an n-propyl group, an n-butyl group, an *n*-pentyl group, an *n*-hexyl group, an *n*-heptyl group, an *n*-octyl group, an *n*-nonyl group, an *n*-decyl group, or an *n*-undecyl group. Examples of the aryl group include a phenyl group, a biphenyl group, and a naphthyl group, among which a phenyl group is preferred. Examples of the substituent of the aryl group include a hydroxy group; halogen atoms such as a fluorine atom, a chlorine atom, and a bromine atom; and linear or branched alkyl group having from 1 to 4 carbon atoms, such as a methyl group, an ethyl group, an n-propyl group, an isopropyl group, an *n*-butyl group, a *tert*-butyl group, and an isobutyl group.

Specific examples of the hydrazide compounds represented by Formula (1) include lauric acid hydrazide, salicylic acid hydrazide, formhydrazide, acetohydrazide, propionic acid hydrazide, *p*-hydroxybenzoic acid hydrazide, naphthoic acid hydrazide, and 3-hydroxy-2-naphthoic acid hydrazide.

Examples of the above-described dihydrazide compounds include dihydrazide compounds represented by the following Formula (2):

H₂NHN-X-NHNH₂ (2)

(wherein, X represents a -CO- group or a -CO-A-CO- group; and A represents an alkylene group optionally having a substituent, or an arylene group optionally having a substituent).

In Formula (2), the alkylene group represented by A may be a divalent aliphatic hydrocarbon group having from 1 to 20 carbon atoms and is preferably a linear alkylene group having from 1 to 12 carbon atoms, such as a methylene group, an ethylene group, a trimethylene group, a tetramethylene group, a pentamethylene group, a hexamethylene group, a heptamethylene group, an octamethylene group, a nonamethylene group, a decamethylene group, or an undecamethylene group. Examples of the substituent of the alkylene group include a hydroxy group.

Examples of the arylene group include a phenylene group, a biphenylene group, a naphthylene group, an anthrylene group, and a phenanthrylene group, among which a phenylene group and a naphthylene group are preferred. Examples of the substituent of the arylene group include a hydroxy group; halogen atoms such as a fluorine atom, a chlorine atom, and a bromine atom; and linear or branched alkyl group having from 1 to 4 carbon atoms, such as a methyl group, an ethyl group, an *n*-propyl group, an isopropyl group, an *n*-butyl group, a *tert*-butyl group, and an isobutyl group.

Specific examples of the dihydrazide compounds represented by Formula (2) include dibasic acid dihydrazides, such as oxalic acid dihydrazide, malonic acid dihydrazide, succinic acid dihydrazide, adipic acid dihydrazide, azelaic acid dihydrazide, sebacic acid dihydrazide, dodecanoic acid dihydrazide, maleic acid dihydrazide, fumaric acid dihydrazide, diglycolic acid dihydrazide, tartaric acid dihydrazide, malic acid dihydrazide, isophthalic acid dihydrazide, terephthalic acid dihydrazide, dimer acid dihydrazide, and 2,6-naphthoic acid dihydrazide. Thereamong, adipic acid dihydrazide is preferred.

Examples of the above-described polyhydrazide compounds include polyacrylic acid hydrazide.

The hydrazide compounds according to the invention are preferably dihydrazide compounds, more preferably dibasic acid dihydrazides, particularly preferably adipic acid dihydrazide.

Examples of the above-described aminoguanidine compounds include aminoguanidine sulfate, diaminoguanidine sulfate, aminoguanidine hydrochloride, diaminoguanidine hydrochloride, and triaminoguanidine hydrochloride. These compounds may be used singly, or in combination of two or more thereof. As aminoguanidine salts, aminoguanidine sulfate and aminoguanidine hydrochloride are particularly preferred.

The above-described polyamines are not particularly restricted as long as they have two or more amino groups in each molecule and at least one of the amino groups is a primary amino group. In the invention, the polyamines are preferably in a liquid state at normal temperature. In the invention, polyamines having two or more primary amino groups in each molecule are more preferred, and lower aliphatic polyamines having not more than 10 carbon atoms are still more preferred. For example, polyamines represented by the following Formula (3) are particularly preferred since they not only are liquid at normal temperature but also have a high decomposition temperature and a high boiling point and are highly reactive with ketone-based gases.

H₂N-(CH₂CH₂-NH)ₙ-CH₂CH₂NH₂ (3)

(wherein, n is an integer from 0 to 3)

The polyamines represented by Formula (3) are ethylene diamine, diethylene triamine, triethylene tetramine, and tetraethylene pentamine.

Further, in the invention, a polyamine represented by the following Formula (4) can be used.

H₂N-R²-NH₂ (4)

(wherein, R² represents a linear or branched divalent hydrocarbon group having 3 or more carbon atoms)

Examples of the polyamine represented by Formula (4) include lower aliphatic polyamines, such as trimethylene diamine, propylene diamine, and tetramethylene diamine.

In the invention, the above-described polyamines may be used singly, or in combination of two or more thereof. By using high-boiling-point triethylenetetramine (boiling point: 278°C) and/or tetraethylenepentamine (boiling point: 330°C), a gas absorbent having markedly improved heat resistance can be provided.

As described above, the primary amine compound according to the invention has solid or liquid properties. When the primary amine compound is solid, it may be in the form of an aggregate, powder or the like. Meanwhile, when the primary amine compound is liquid, it is preferably adhered to the surface of other material.

In the invention, a preferred mode of the ketone-based gas adsorbent is a complex in which the primary amine compound is supported on an inorganic powder. The mass ratio of the primary amine compound and the inorganic powder is preferably from 5:95 to 60:40, more preferably from 5:95 to 50:50, still more preferably from 5:95 to 40:60.

The inorganic powder used in the invention is not particularly restricted in terms of its component and shape as long as the inorganic powder can be mixed with the primary amine compound. Examples of the inorganic powder include silicate compounds, tetravalent metal phosphate compounds, silica gels, zeolites, micas, hydrotalcites, sepiolites, attapulgites, and bentonites. Thereamong, for example, silicate compounds, tetravalent metal phosphate compounds, silica gels and mica are preferred since they can improve the adsorption performance. Further, in the invention, the inorganic powder is preferably adjusted such that it yields a pH in a range of from 2.0 to 8.0 when dispersed in purified water at a concentration of 5% by mass, and examples of such inorganic powder before the pH adjustment include mica, hydrotalcite, sepiolite, attapulgite, bentonite, and zeolite Y-type. An acid used for the pH adjustment is preferably an inorganic acid, more preferably sulfuric acid or phosphoric acid.

The above-described silicate compounds are not particularly restricted; however, aluminum silicates and magnesium silicates are preferred. In the invention, from the standpoint of water resistance, amorphous aluminum silicate and amorphous magnesium silicate are more preferred. Further, amorphous aluminum silicate is particularly preferred since its complex with a primary amine compound exhibits high ketone adsorption performance under a high-temperature atmosphere. These silicate compounds may be either natural products or synthetic products. The silicate compounds are preferably those which yield a pH of from 2.0 to 8.0 when dispersed in purified water at a concentration of 5% by mass.

For example, synthetic aluminum silicates are represented by the following Formula (5):

xNa₂O·Al₂O₃·ySiO₂·nH₂O (5)

(wherein, x is from 0.1 to 0.5, y is from 5 to 15, and n is from 0.3 to 15).
Magnesium silicates are represented by the following Formula (6):

MgO·zSiO₂·mH₂O (6)

(wherein, z is a positive number of 1 or larger; preferably, z is from 1 to 20 and m a positive number of from 0.1 to 20; more preferably, z is from 1 to 15 and m is from 0.3 to 10; particularly preferably, z is from 3 to 15 and m is from 1 to 8).

Synthetic silicate compounds can be those which are obtained by, for example, mixing an aqueous solution of an aluminum salt or a magnesium salt with an aqueous solution of an alkali metal silicate, adding an acid or an alkali to the resulting mixture as required at room temperature under an atmospheric pressure, allowing the resultant to coprecipitate while maintaining the pH at about 3 to about 7, and subsequently aging the resulting coprecipitate at a temperature of, for example, from about 40°C to about 100°C, or washing the coprecipitate with water and then dehydrating and drying the coprecipitate without aging.

In the production of a synthetic aluminum silicate, the amount of an aluminum water-soluble salt to be used and that of an alkali metal silicate to be used are selected such that a molar ratio SiO₂/Al₂O₃ of 6 or higher, preferably in a range of from 6 to 50, more preferably in a range of from 8 to 15, is attained.

In the production of a synthetic magnesium, the amount of a magnesium water-soluble salt to be used and that of an alkali metal silicate to be used are selected such that a molar ratio SiO₂/MgO of 1 or higher, preferably in a range of from 1 to 20, more preferably in a range of from 1 to 15, is attained.

As other method of producing a synthetic silicate compound, for example, a method in which an aluminum or magnesium water-soluble salt is added to a silica sol, the resultant is then sufficiently and uniformly mixed while maintaining the pH of the system at about 3 to 7 with an acid or an alkali, and the resulting mixture is further heated to, for example, about 40°C to about 100°C, allowed to age and subsequently washed with water, dehydrated and dried, can be employed. It is noted here that the aging process is not necessarily required. Further, the amount of the silica sol to be used and that of the aluminum or magnesium water-soluble salt to be used can be selected in the same manner to attain the above-described molar ratio of SiO₂/Al₂O₃ or SiO₂/MgO. This production method is also capable of synthesizing a compound containing both metals from a mixed aqueous solution of an aluminum water-soluble salt and a magnesium water-soluble salt, rather than individually synthesizing amorphous aluminum silicate or amorphous magnesium silicate.

The above-described tetravalent metal phosphate compounds are preferably insoluble or hardly soluble in water. Specific examples of such tetravalent metal phosphate compounds include zirconium phosphate, titanium phosphate, and tin phosphate. These compounds may be crystalline having various crystal systems (e.g., *α*-type crystal, *β*-type crystal, *γ*-type crystal, and NASICON-type crystal) or amorphous, and any of these compounds can be used. In the invention, an *α*-type crystalline compound is preferred since it is highly resistant to water and its complex with a primary amine compound exhibits high ketone-based gas adsorption performance under a high-temperature atmosphere. Further, the above-described tetravalent metal phosphate compounds are preferably those which yield a pH of from 2.0 to 7.0 when dispersed in purified water at a concentration of 5% by mass.

As the above-described silica gels, those whose surface area or pore size is adjusted by a production method and which have various characteristics can be used. One example of a method of producing such a silica gel is a method of washing a gel, which is obtained by adding sulfuric acid to liquid glass, with water and subsequently drying and pulverizing the thus washed gel. The silica gels are preferably compounds which yield a pH of from 2.0 to 7.0 when dispersed in purified water at a concentration of 5% by mass.

The above-described zeolites may be either natural products or synthetic products. Zeolites have various structures, and any known zeolite can be used. Examples of the zeolites include A-type, X-type, Y-type, *α*-type, *β*-type and ZSM-5 zeolites. The zeolites are preferably compounds which yield a pH of preferably from 2.0 to 9.0, more preferably from 2.0 to 8.0, when dispersed in purified water at a concentration of 5% by mass. When the pH of the dispersion is outside the range of from 2.0 to 9.0, the pH is preferably adjusted to be in this range.

The BET specific surface area of the above-described inorganic powder is preferably not less than 80 m²/g, more preferably from 100 to 700 m²/g.

The shape and size of the inorganic powder is not particularly restricted; however, the average particle size is preferably from 0.1 to 20 µm, more preferably from 1.0 to 10 µm.

A method of allowing the above-described primary amine compound to be supported on the inorganic powder will now be described. The inorganic powder or a dispersion thereof is mixed with the primary amine compound or a solution or dispersion thereof, and the resulting mixture is subsequently dried or heated as required, whereby a complex can be obtained. Alternatively, a complex can also be obtained by dropwisely adding or spraying a solution of the primary amine compound to the inorganic powder while stirring the inorganic powder in a temperature range of from room temperature to about 60°C, and subsequently drying or heating the resultant as required. The dispersion of the inorganic powder and the solution of the primary amine compound may be aqueous solutions or solutions of an organic solvent (e.g., an alcohol); however, they are preferably aqueous solutions.

Here, from the standpoint of the ketone-based gas adsorption effect, the ratio of the amount of the inorganic powder to be used and that of the primary amine compound to be used is as follows. The primary amine compound is used in an amount of preferably from 5 to 200 parts by mass, more preferably from 10 to 100 parts by mass, still more preferably from 15 to 50 parts by mass, with respect to 100 parts by mass of the inorganic powder.

In cases where drying is performed in the above-described production method, the drying temperature is preferably from 60°C to 140°C, more preferably from 80°C to 120°C, and the drying may be performed under reduced pressure. The treatment time of this drying step may be set depending on the conditions since the optimum treatment time varies depending on the drying temperature, treatment amount and apparatus.

After the drying step, a heat treatment may be performed at a temperature of from 80°C to 240°C, preferably from 90°C to 220°C, so as to improve the water resistance of the resulting gas adsorbent composed of the complex. In cases where the primary amine compound contains aminoguanidine hydrochloride, the heating temperature is preferably from 110°C to 150°C, more preferably from 120°C to 130°C. Further, the duration of the heat treatment may be set depending on the conditions since the optimum duration varies depending on the drying temperature, treatment amount and apparatus. For the production of the ketone-based gas adsorbent of the invention, the drying step and the heat treatment may be performed in combination.

As described above, the ketone-based gas adsorbent of the invention may consist of only a primary amine compound, or may be a complex composed of a primary amine compound and other material (e.g., inorganic powder). In either case, the ketone-based gas adsorbent of the invention is preferably in a powder form and, from the standpoint of the ketone-based gas adsorption effect, the average particle size thereof is preferably from 0.01 to 50 µm, more preferably from 0.02 to 20 µm.

The ketone-based gas adsorbent of the invention is also characterized by having high ketone-based gas adsorption performance under a high-temperature atmosphere. The "adsorption performance under a high-temperature atmosphere" means that, for example, when a fiber, a resin molded article or the like containing the ketone-based gas adsorbent of the invention is heated, the generation of a ketone-based gas therefrom can be inhibited. The ketone-based gas adsorbent of the invention is capable of adsorbing ketone-based gases through chemical adsorption and maintaining the adsorbed state even at a temperature of, for example, from 40°C to 90°C. Therefore, the ketone-based gases once adsorbed are not released.

The ketone-based gas adsorbent of the invention can be used in combination with other ketone-based gas adsorbent(s), such as activated charcoal, zeolite and various plant extracts.

Further, the ketone-based gas adsorbent of the invention can also be used in combination with an adsorbent that adsorbs a gas other than ketone-based gases.

The gas adsorbent composition of the invention (hereinafter, referred to as "gas adsorbent-containing composition") contains: the above-described ketone-based gas adsorbent of the invention; and at least one gas adsorbent selected from the group consisting of basic gas adsorbents, sulfur-based gas adsorbents and organic acidic gas adsorbents. The properties of the gas adsorbent-containing composition of the invention are not particularly restricted, and the composition may be solid or liquid; however, when the composition is composed of the ketone-based gas adsorbent and at least one gas adsorbent selected from the group consisting of basic gas adsorbents, sulfur-based gas adsorbents and organic acidic gas adsorbents, the composition is preferably a solid mixture.

The basic gas adsorbents are adsorbents that adsorb basic gases, such as ammonia and trimethylamine. Examples of the basic gas adsorbents include tetravalent metal phosphate compounds that are insoluble or hardly soluble in water. Preferred specific examples of the tetravalent metal phosphate compounds include zirconium phosphate, titanium phosphate, and tin phosphate. These compounds may be crystalline having various crystal systems (e.g., *α*-type crystal, *β*-type crystal, *γ*-type crystal, and NASICON-type crystal) or amorphous.

The sulfur-based gas adsorbents are adsorbents that adsorb sulfur-based gases, such as hydrogen sulfide and methyl mercaptan. Examples of the sulfur-based gas adsorbents include tetravalent metal phosphate compounds supporting at least one metal ion selected from copper, zinc and manganese ions; zinc oxide; copper silicate; and zinc silicate. The metal ion supported on the tetravalent metal phosphate compounds is particularly preferably a copper ion since a high adsorption effect for hydrogen sulfide and the like is attained.

For the preparation of a tetravalent metal phosphate compound having a metal ion supported thereon, the tetravalent metal phosphate compound may be brought into contact with a salt solution of the metal ion, and the metal ion may be supported by ion exchange or the like. The amount of the metal ion to be supported can be freely adjusted as desired within the ion exchange capacity of the tetravalent metal phosphate compound up to 100%.

Further, with regard to zinc oxide, copper silicate and zinc silicate, ones having a large specific surface area are preferred since they have high adsorption performance.

The organic acidic gas adsorbents are adsorbents that adsorb acetic acid, isovaleric acid, butyric acid and the like. Examples of the organic acidic gas adsorbents include hydrated zirconium oxide and hydrated titanium oxide.

Hydrated zirconium oxide can be prepared by hydrolyzing a zirconium-containing solution, such as an aqueous zirconium oxychloride solution, with water or an alkaline solution. It is noted here that various names, such as "zirconium oxyhydroxide", "zirconium hydroxide", "hydrous zirconium oxide" and "zirconium oxide hydrate", are used for hydrated zirconium oxide; however, all of these names mean the same compound.

In the gas adsorbent-containing composition of the invention, the content ratio of the ketone-based gas adsorbent and the at least one gas adsorbent selected from the group consisting of basic gas adsorbents, sulfur-based gas adsorbents and organic acidic gas adsorbents is not particularly restricted and, when the total content thereof is taken as 100%, the content of the ketone-based gas adsorbent and that of the at least one gas adsorbent are preferably from 40 to 90% by mass and from 10 to 60% by mass, more preferably from 60 to 80% by mass and from 20 to 40% by mass, respectively. It is noted here that the lower limit of the content of the primary amine compound is preferably 5% by mass, more preferably 10% by mass, with respect to the whole composition.

The gas adsorbent components contained in the gas adsorbent-containing composition of the invention are all preferably in a powder form. From the standpoint of the effect of adsorbing ketone-based gases and the like, the average particle size of the gas adsorbent components is preferably from 0.01 to 50 µm, more preferably from 0.02 to 20 µm. The gas adsorbent-containing composition containing such gas adsorbent components having the above-described average particle size can be used in both powder paints and liquid paints.

Depending on the intended purpose of use, the gas adsorbent or gas adsorbent-containing composition of the invention may be granulated. As a method of producing a granule, any conventionally known powder granulation method can be employed. For example, a method of producing a granule using alumina sol, clay or the like as a binder can be employed. The particle size can be adjusted as appropriate depending on the hardness and density of the resulting granule, the grinding strength and the like, and it is preferably from 0.1 to 3 mm from the standpoint of the ease of handing.

Further, the gas adsorbent or gas adsorbent-containing composition of the invention can be accommodated in a capsule to obtain a deodorant product, although the size thereof is not restricted.

The gas adsorbent and gas adsorbent-containing composition of the invention are effective for deodorization of tobacco odor, household odor, body odor, urine and fecal odor, waste odor and the like.

In the invention, the gas adsorbent or gas adsorbent-containing composition in a powder, granule or particle form can be packed into, for example, a cartridge to obtain a deodorant product.

In addition to the above-described gas adsorbent components, the gas adsorbent-containing composition of the invention may further contain various additives, such as an antiseptic agent, a suspending agent, a pH modifier, an antibacterial agent, a flavoring agent, an anionic surfactant, a cationic surfactant, a nonionic surfactant, an amphoteric surfactant, a coloring agent, a clarifying agent, a viscosity modifier, and an essential oil.

Examples of the antiseptic agent include benzoic acid, sorbic acid, propionic acid, dehydroacetic acid, and salts thereof; organic acid esters, such as butyl *p*-oxybenzoate and isobutyl *p*-oxybenzoate; inorganic salts, such as sodium sulfite, sodium hyposulfite, and sodium pyrosulfite; and plant-derived components such as pectin extract.

Examples of the suspending agent include powders made of silicon dioxide, magnesium stearate or the like; surfactants, such as polyoxyethylene hydrogenated castor oil and polyoxyethylene sorbitan monostearate; and gelling agent, such as xanthan gum and polyethylene glycol.

Examples of the pH modifier include citric acid, fumaric acid, DL-maleic acid, and salts thereof; carbonates, such as sodium bicarbonate and potassium carbonate; phosphoric acid; and phosphates, such as sodium dihydrogen phosphate.

Examples of the antibacterial agent include silver-based inorganic antibacterial agents, trichlorocarbanilide, benzalkonium chloride, benzethonium chloride, halocarban, chlorhexidine hydrochloride, dihydrofarnesol, and isopropylmethylphenol.

The gas adsorbent-containing composition of the invention may contain a gelling agent. Examples of the gelling agent include seaweed polysaccharides, such as carrageenan and sodium alginate; seed polysaccharides, such as guar gum and tamarind seed gum; plant sap polysaccharides, such as gum arabic and tragacanth gum; fermentation polysaccharides, such as xanthan gum and gellan gum; proteins such as gelatin; and cellulose derivatives such as carboxymethyl cellulose.

When the gas adsorbent-containing composition of the invention contains a gelling agent, a solid or semi-solid deodorant product can be obtained.

A gas adsorbent-containing dispersion, which is one of the raw materials for the production of a deodorant processed article, can be prepared using the gas adsorbent or gas adsorbent-containing composition of the invention. Examples of a method of producing the gas adsorbent-containing dispersion include a method of directly dispersing the gas adsorbent or the gas adsorbent-containing composition in a dispersion medium, and a method of dispersing the gas adsorbent or the gas adsorbent-containing composition in a dispersion medium using a dispersant. In cases where the gas adsorbent of the invention is a complex, a method of preparing a dispersion of the primary amine compound and a dispersion of the inorganic powder used as a carrier and then mixing these dispersions can be employed. The average particle size of the gas adsorbent or gas adsorbent-containing composition used in this case is preferably from 0.01 to 50 µm since reaggregation is inhibited and excellent dispersibility is attained. This gas adsorbent-containing dispersion may contain an antifoaming agent, an antiseptic agent, a viscosity modifier and/or the like as required. For the production of the gas adsorbent-containing dispersion, for example, a sand mill, a disper or a ball mill can be employed.

As the dispersion medium, any dispersion medium can be used with no restriction as long as it is soluble in water and hydrophilic. Specific examples thereof include protic solvents, such as water, alcohols (e.g., ethanol and glycerin) and alkylene glycols; and aprotic solvents, such as dimethylformamide, dimethylacetamide, dimethylsulfoxide, tetrahydrofuran, and acetone. These protic solvents and aprotic solvents may be used singly or in combination. The dispersion medium is preferably water or an alcohol, more preferably water.

The dispersant is not particularly restricted. Examples of the dispersant include anionic surfactants, such as alkenyl succinates, alkylbenzene sulfonates, alkylnaphthalene sulfonates, alkyl sulfates, higher alcohol sulfates, polyoxyethylene alkyl ether sulfates, dialkyl sulfosuccinates, alkyl phosphates, phosphate-based copolymers, and polycarboxylic acid-type polymeric surfactants; nonionic surfactants, such as polyoxyethylene alkyl ethers, polyoxyethylene alkyl allyl ethers, and organically modified organopolysiloxanes; cationic surfactants, such as alkylamine salts and quaternary ammonium salts; betaine-type amphoteric surfactants, such as alkyl betaines and amide betaine; pyrophosphates; tripolyphosphates; and polyamines such as triethanolamine. These dispersants may be used singly, or in combination of two or more thereof.

As the dispersant, one having an acidic functional group, such as a carboxyl group, a sulfone group or a phosphate group, is preferred. In this case, the dispersant may have a surfactant effect. Further, a surfactant having an acidic functional group and a nonionic dispersant may be used in combination as well.

The dispersant having an acidic functional group is more preferably an acidic functional group-containing copolymer. The basic skeleton of this copolymer can be constituted by an ester chain, a vinyl chain, an acrylic chain, an ether chain, a urethane chain or the like. The dispersant may also be a polymer in which some of the hydrogen atoms in the molecule are substituted with halogen atoms. Thereamong, acrylic resins, polyester resins and alkyd resins are preferred, and acrylic resins and polyester resins are particularly suitable. The acidic functional group contained in the polymer is preferably a phosphate group. The acidic functional group may be randomly arranged in the resin molecule; however, the acidic functional group is preferably arranged at a terminal of the molecule depending on a block or graft structure. The reason for this is because, when the gas adsorbent adsorbs thereto, the adsorbent is likely to assume a dispersion-stabilized structure due to solvation. Examples of a counter cation include alkali metal salts, ammonium salts, and amine salts. Thereamong, alkyl ammonium salts are particularly preferred.

The acid value of the dispersant having an acidic functional group is preferably from 5 to 150 mg KOH/g, more preferably from 30 to 130 mg KOH/g. With the acid value of the dispersant being in a range of from 5 to 150 mg KOH/g, the dispersant favorably adsorbs to the adsorbent particle surface, so that excellent dispersion stability is attained.

The weight-average molecular weight of the dispersant having an acidic functional group is preferably from 800 to 100,000, more preferably from 800 to 10,000. With the weight-average molecular weight being in a range of from 800 to 100,000, a favorable dispersion effect is obtained, and dispersoid aggregation and an increase in viscosity of the resulting dispersion are inhibited.

As the dispersant having an acidic functional group, a commercial product, examples of which include "DISPERBYK-110, 170, 180, and 190" (trade names) manufactured by BYK-Chemie GmbH, "SER-AD FA192" (trade name) manufactured by Servo Delden BV, "SOLSPERSE 3000, 9000, 13240, 13940, 17000, 17240, 17940, 21000, 24000, 26000, and 27000" (trade names) manufactured by Zeneca Colors, "FLOWLEN G-700" (trade name) manufactured by Kyoeisha Chemical Co., Ltd. and "AJISPER PA111" (trade name) manufactured by Ajinomoto Co., Inc., can be used.

The amount of the dispersant to be used for the production of the gas adsorbent-containing dispersion is, from the standpoint of dispersibility, preferably from 0.1 to 15 parts by mass, more preferably from 0.5 to 12 parts by mass, particularly preferably from 1 to 10 parts by mass, with respect to 100 parts by mass of the gas adsorbent or the gas adsorbent-containing composition.

As the above-described antifoaming agent, any of a foam-breaking antifoaming agent, a foam-suppressing antifoaming agent and a defoaming antifoaming agent may be used. Examples of the foam-breaking antifoaming agent include polysiloxane solutions.

The above-described viscosity modifier is not particularly restricted as long as it is capable of adjusting the viscosity of a dispersion. Examples of the viscosity modifier include cellulose-based thickening agents, such as methyl cellulose, carboxymethyl cellulose, methylhydroxycellulose, methylhydroxypropyl cellulose and hydroxyethyl cellulose; natural polysaccharides, such as gum arabic, tragacanth gum and guar gum; various polyacrylamide-based polymers; polyethylene oxides; and polyvinyl alcohols.

The solid content of all gas adsorbents in the gas adsorbent-containing dispersion is, from the standpoint of dispersibility, preferably from 1 to 60% by mass, more preferably from 3 to 40% by mass, still more preferably from 5 to 25% by mass. When the solid content is from 1 to 60% by mass, the gas adsorbent-containing dispersion has favorable handling properties and can be used as, for example, a spray material, a lotion material or a milky liquid; therefore, the gas adsorbent-containing dispersion is suitable as a cosmetic material or the like. Further, the gas adsorbent-containing dispersion is suitably used for preparing the below-described gas adsorbent-containing processed liquid and the like.

A gas adsorbent-containing processed liquid, which is a paint composition (liquid paint) used for obtaining a deodorant processed article by coating a substrate or the like with the paint composition, can be prepared by incorporating a binder into the gas adsorbent-containing dispersion. The binder is not particularly restricted, and any binder (e.g., an alkyd resin, an amino alkyd resin, an acrylic resin, a vinyl chloride resin, a silicone resin, a fluorocarbon resin, an epoxy resin, a urethane resin, or a saturated polyester resin) that is generally used for surface treatment of, for example, a fiber (e.g., an acrylic fiber or a urethane-based fiber), a sheet containing the fiber (e.g., a nonwoven fabric or a woven fabric), or a resin-made sheet or plate. The binder may be of a type that is made into a coating film by an arbitrary mechanism and, when the coating film is to be cured, the binder may be of an oxidative polymerization-type, a wet polymerization-type, a heat curing-type, a catalyst curing-type, a UV curing-type, a polyol curing-type or the like. The binder is preferably used such that the total amount of the binder and the solids of the gas adsorbent components in the dispersion is from 5 to 50% by mass with respect to the resulting gas adsorbent-containing processed liquid.

Further, from the standpoints of the adhesiveness to a substrate or the like and the adsorptivity of ketone-based gases and the like, the content ratio between the solids of the gas adsorbent components and the binder in the gas adsorbent-containing processed liquid is preferably from 10 to 300 parts by mass of the binder with respect to 100 parts by mass of the solids of the gas adsorbent components. With the content ratio of the binder being from 10 to 300 parts by mass, a sufficient fixation strength is attained when the gas adsorbent-containing processed liquid is spread on a fiber, a nonwoven fabric, a sheet or the like, so that detachment of the gas adsorbent components does not occur. In addition, a reduction in adsorption performance caused by coating of the gas adsorbent components with the binder does not occur as well.

The gas adsorbent-containing processed liquid can be easily prepared by a method of mixing the raw material components using a commonly used apparatus, such a ball mill, a roll mill, a disper or a mixer.

In the invention, examples of a deodorant processed article obtained by coating or the like of the gas adsorbent-containing processed liquid include deodorant fibers, deodorant sheets (including deodorant films), clothings having a deodorizing function, deodorant molded articles (e.g., plates, container, lids, and casings), and deodorant walls. These deodorant processed articles have a coating film obtained by coating the surface of a substrate with the gas adsorbent-containing processed liquid and then drying the resultant.

In the case of a deodorant sheet, a pre-processed raw material sheet is not particularly restricted, and the material thereof may be selected in accordance with the intended use and the like and can be an organic material (e.g., a resin or paper), an inorganic material, or a complex thereof. As for the constitution of the raw material sheet, for example, the air permeability from one side to the other side is not particularly restricted. Preferred specific examples of the raw material sheet include Japanese paper, synthetic paper, nonwoven fabrics, and resin films, and the raw material sheet is particularly preferably a sheet of paper made of natural pulp and/or synthetic pulp. When natural pulp is used, since gas adsorbent particles are easily sandwiched between finely branched fibers, a practical support can be obtained. Meanwhile, a synthetic pulp is advantageous in that it has excellent chemical resistance. A sheet of paper having various adjusted properties can be obtained by using natural pulp and synthetic pulp at an appropriate mixing ratio and, generally speaking, a sheet of paper having excellent strength, water resistance, chemical resistance, oil resistance and the like can be obtained by increasing the ratio of the synthetic pulp, whereas a sheet of paper having excellent water adsorption, gas permeability, hydrophilicity, moldability, texture and the like can be obtained by increasing the ratio of the natural pulp.

The deodorant sheet may be one in which the gas adsorbent components contained in the gas adsorbent-containing composition are incorporated in the entirety of the raw material sheet from one side to the other side, or in a surface layer of the raw material sheet on one side or the other side, or inside of the raw material sheet excluding the surface layer.

The amount of the gas adsorbent components supported in the deodorant sheet is not particularly restricted. Generally speaking, an increase in the amount of the supported gas adsorbent components allows the deodorizing properties to be strongly exhibited and maintained for a prolonged period; however, an increase to a certain level or higher does not lead to a large difference in deodorizing effect. Therefore, the amount of the supported gas adsorbent components is preferably from 0.1 to 10 parts by mass per 100 parts by mass of the raw material sheet.

The deodorant sheet can be used as, for example, a cosmetic paper, a medical package paper, a food package paper, an electrical equipment packing paper, a nursing care paper product, a freshness-keeping paper, a paper cloth, an air-purifying filter, a wall paper, a tissue paper, or a sanitary paper.

A method of producing a deodorant sheet using the gas adsorbent-containing processed liquid is not particularly restricted. For example, a base paper or a fiber sheet can be coated with, immersed in, or sprayed with the gas adsorbent-containing processed liquid. When the gas adsorbent-containing processed liquid is used, it is preferably applied such that the gas adsorbent components are supported in an amount of about 0.05 to 10 g/m².

In the case of producing a deodorant sheet without using the gas adsorbent-containing processed liquid, for example, a method of adhering an inorganic powder constituting the gas adsorbent of the invention to a base paper or a fiber sheet and subsequently spraying or the like a primary amine compound solution thereto, or a method of performing a paper-making process using a slurry that contains the gas adsorbent-containing composition and a pulp, can be adopted. In the latter case, a deodorant sheet in which gas adsorbent components are supported on a sheet of paper can be obtained by preparing a slurry that contains the gas adsorbent-containing composition and a pulp at a prescribed ratio; subsequently adding a cationic or anionic coagulant to the thus obtained slurry in an amount of 5% by mass or less to generate aggregates; preparing a sheet of paper using the thus obtained aggregates in accordance with a known method; and then drying the thus obtained sheet of paper at a temperature of, for example, from 100°C to 190°C.

In the deodorant processed article of the invention, as a mode other than the one composed of a coating film, the gas adsorbent components may be integrated with a substrate. That is, a deodorant product in which the gas adsorbent components are contained in a substrate composed of an inorganic material or organic material can be used as the deodorant processed article.

The deodorant processed article of the invention is preferably a resin molded article or foam-molded article in which a substrate composed of an organic material is used. For the production of such a resin molded article, a gas adsorbent-containing composition which contains a resin as a molding material is used. When this resin is a thermoplastic resin, the gas adsorbent-containing composition may be a mixture or melt-kneaded product that contains the thermoplastic resin and a gas adsorbent component. The resin molded article can be produced by loading the gas adsorbent-containing composition into a molding machine. Alternatively, a resin molded article can also be produced by preparing a pellet-form resin using a gas adsorbent-containing composition that contains a gas adsorbent component at a high concentration, subsequently mixing this resin with a main resin, and then loading the resulting mixture to a molding machine. It is noted here that, in the gas adsorbent-containing composition, an additive(s) such as a pigment, a dye, an antioxidant, a light stabilizer, an antistatic agent, a foaming agent, an impact resistance enhancer, a glass fiber, a desiccant and/or a bulking agent may also be incorporated as required for the purpose of, for example, improving the physical properties. As a molding method for the production of the above-described resin molded article or foam-molded article, any conventionally known molding method, such as injection molding, extrusion molding, inflation molding, vacuum molding or foam molding, can be adopted.

Examples of the deodorant processed article containing an organic material include casings of household electric appliances (e.g., air purifiers and refrigerators), general household goods (e.g., trash boxes and drainers), nursing care products (e.g., portable toilets), furnitures, beddings, deodorant fibers, deodorant sheets (including deodorant films), other deodorant molded articles (e.g., plates, container, lids, and casings), and deodorant walls.

Examples of a deodorant processed article containing the gas adsorbent or gas adsorbent-containing composition of the invention include deodorant fibers, deodorant paints, deodorant sheets, and deodorant resin molded articles.

The deodorant fibers containing the gas adsorbent or gas adsorbent-containing composition of the invention can be used in a wide variety of fiber products, such as clothings, underwear, stockings, socks, futons, futon covers, cushions, blankets, carpets, curtains, sofas, covers, sheets, car seats, car mats, and air filters. As a method of adding the deodorant fibers to a fiber product, for example, a method of spreading the fibers on the front surface or back side of the fiber product using a binder or a method of kneading the fibers into a fiber resin can be employed. Further, the deodorant paints containing the gas adsorbent or gas adsorbent-containing composition of the invention can be used on, for example, interior walls and exterior walls of buildings, and interior walls of railway vehicles. Moreover, the deodorant sheets containing the gas adsorbent or gas adsorbent-containing composition of the invention can be used as, for example, medical package papers, food package papers, freshness-keeping papers, paper clothings, air-purifying filters, wall papers, tissue papers, sanitary papers, nonwoven fabrics, papers, fibers, and films.

Furthermore, the deodorant resin molded articles containing the gas adsorbent or gas adsorbent-containing composition of the invention can be used in, for example, household electric appliances (e.g., air purifiers and refrigerators), general household goods (e.g., trash boxes and drainers), nursing care products (e.g., portable toilets), and daily commodities.

The gas adsorbent or gas adsorbent-containing composition of the invention and deodorant processed articles containing the same can also be used in clothings (e.g., underwear, stockings, and caps) as a means for inhibiting the generation of an odor therefrom or masking a generated odor when body odor or menstrual odor adheres to the clothings.

Further, by using the gas adsorbent or gas adsorbent-containing composition of the invention in those materials which generate a ketone-based gas from their substrates (e.g., building materials, such as plywood boards, laminated wood materials, flooring materials, particle boards and heat insulating materials; floor carpets; noise reduction pads; cushion materials; car seats; headrests; armrests; door trims; molded ceilings; sun visors; rear package trays; instrument panels; and dash insulator), volatilization of a ketone-based gas from the substrates themselves can be reduced.

### EXAMPLES

The invention will now be described more concretely by way of examples thereof; however, the invention is not restricted to thereto. It is noted here that "%" means "% by mass".

### 1. Gas Adsorbent Evaluation Methods

### (1) Median Particle Size (d50)

Each subject adsorbent was measured using a laser diffraction-type particle size distribution analyzer "MS2000" (model name) manufactured by Malvern Instruments, Ltd., and the median particle size was analyzed based on volume. It is noted here that the content ratio (%) of the particle size distribution is based on "% by volume" in total particles determined by this analysis method; however, since the density of the measured powders is constant, the content ratio (%) has the same meaning as "% by mass".

### (2) BET Specific Surface Area

The BET specific surface area was measured in accordance with JIS Z8830 "Determination of the specific surface area of powders (solids) by gas adsorption" (2001 edition) using a continuous flow-type surface area analyzer "SA-6200" (model name) manufactured by HORIBA, Ltd.

### (3) Adsorption Capacity

The subject adsorbent powder in an amount of 0.01 g was placed in a 5-L test bag made of a vinyl alcohol-based polymer film, and 3 L of vaporized acetone, methyl ethyl ketone or diacetyl (initial concentration: 300 ppm) was injected thereto. After storing the test bag at 20°C for 1 hour, the residual gas concentration in the test bag was measured using a gas detection tube. The adsorption capacity was indicated as the volume of the gas adsorbed per 1 g of the sample.

### 2. Production and Evaluation of Gas Adsorbents (1)

### Example 1-1

An aqueous solution obtained by dissolving 3 g of white aminoguanidine sulfate in 10 mL of ion exchanged water was sprayed to 10 g of white amorphous aluminum silicate particles having a specific surface area of 605 m²/g and an average particle size of 6 µm, and the resultant was subsequently mixed at room temperature for 20 minutes.

It is noted here that the pH at which the amorphous aluminum silicate particles were dispersed in purified water at a concentration of 5% by mass was 6.5. Then, the thus obtained sprayed product was heated under vacuum at 100°C for 60 hours, whereby about 13 g of white powder was obtained. Thereafter, the adsorption capacity of this adsorbent composed of the white powder was measured for acetone and diacetyl. The results thereof are shown in Table 1.

### Example 1-2

An aqueous solution obtained by dissolving 2 g of white adipic acid dihydrazide in 10 mL of ion exchanged water was sprayed to 10 g of white ZSM-type zeolite particles having a specific surface area of 388 m²/g and an average particle size of 2 µm, and the resultant was subsequently mixed at room temperature for 20 minutes. It is noted here that the pH at which the zeolite particles were dispersed in purified water at a concentration of 5% by mass was 8.0.

Then, the thus obtained sprayed product was heated at 120°C for 32 hours, whereby about 12 g of white powder was obtained. Thereafter, the adsorption capacity of this adsorbent composed of the white powder was measured for acetone and diacetyl. The results thereof are shown in Table 1.

### Example 1-3

An aqueous solution obtained by dissolving 3 g of diethylenetriamine in 10 mL of ion exchanged water was sprayed to 10 g of white silica gel particles having a specific surface area of 218 m²/g and an average particle size of 12 µm, and the resultant was subsequently mixed at room temperature for 20 minutes. It is noted here that the pH at which the silica gel particles were dispersed in purified water at a concentration of 5% by mass was 6.8. Then, the thus obtained sprayed product was heated at 100°C for 55 hours, whereby about 13 g of white powder was obtained. Thereafter, the adsorption capacity of this adsorbent composed of the white powder was measured for acetone and diacetyl. The results thereof are shown in Table 1.

### Example 1-4

The adsorption capacity of succinic acid dihydrazide powder was measured for acetone and diacetyl. The results thereof are shown in Table 1.

### Comparative Example 1-1

An aqueous solution obtained by dissolving 3 g of white ascorbic acid fatty acid ester in 10 mL of ion exchanged water was sprayed to 10 g of white silica gel particles having a specific surface area of 218 m²/g and an average particle size of 12 µm, and the resultant was subsequently mixed at room temperature for 20 minutes. It is noted here that the pH at which the silica gel particles were dispersed in purified water at a concentration of 5% by mass was 5.6.

Then, the thus obtained sprayed product was heated at 100°C for 5 hours, whereby about 13 g of white powder was obtained. Thereafter, the adsorption capacity of this adsorbent composed of the white powder was measured for acetone and diacetyl. The results thereof are shown in Table 1.

### Comparative Example 1-2

The adsorption capacity of an activated charcoal "TAIKO CW350A" (trade name) manufactured by Futamura Chemical Co., Ltd. was measured for acetone and diacetyl. The results thereof are shown in Table 1.

**(Table 1)**

| | Adsorption capacity (mL/g) | |
|---|---|---|
| | Acetone | Diacetyl |
| Example 1-1 | 13 | 15 |
| Example 1-2 | 20 | 20 |
| Example 1-3 | 12 | 15 |
| Example 1-4 | 7 | 11 |
| Comparative Example 1-1 | 4 | 4 |
| Comparative Example 1-2 | 5 | 7 |

It was confirmed that the adsorbents of Examples 1-1 to 1-4 had greater ketone-based gas adsorption capacity and superior adsorption performance than the adsorbents of Comparative Examples 1-1 and 1-2.

### 3. Production and Evaluation of deodorant processed cloths

### Example 1-5

An adsorbent-containing liquid was obtained by mixing 1 g of the gas adsorbent of Example 1-1 with 3 g of an acrylic binder. Subsequently, a polyester cloth was immersed in this adsorbent-containing liquid such that the gas adsorbent adhered to the polyester cloth in an amount of 2 g/m², after which the polyester cloth was dried at 130°C for 2 minutes to obtain a deodorant processed cloth. The thus obtained deodorant processed cloth was cut into a size of 10 cm × 10 cm and used as a test cloth. Then, this test cloth was placed in a 5-L test bag made of a vinyl alcohol-based polymer film, the test bag was tightly sealed, and 3L of air containing 50 ppm of diacetyl was injected thereto. Thereafter, the test bag was left to stand at 20°C for 2 hours, and the residual gas concentration was measured using a gas detection tube. The result thereof is shown in Table 2.

### Example 1-6

A deodorant processed cloth was produced in the same manner as in Example 1-5, except that the gas adsorbent was changed to the gas adsorbent of Example 1-2. The deodorizing test result is shown in Table 2.

### Example 1-7

A deodorant processed cloth was produced in the same manner as in Example 1-5, except that the gas adsorbent was changed to the gas adsorbent of Example 1-3. The deodorizing test result is shown in Table 2.

### Comparative Example 1-3

A deodorant processed cloth was produced in the same manner as in Example 1-5, except that the gas adsorbent was changed to the gas adsorbent of Comparative Example 1-1. The deodorizing test result is shown in Table 2.

### Comparative Example 1-4

A deodorant processed cloth was produced in the same manner as in Example 1-5, except that the gas adsorbent was changed to the gas adsorbent of Comparative Example 1-2. The deodorizing test result is shown in Table 2.

**(Table 2)**

| | Diacetyl residual concentration (ppm) |
|---|---|
| Example 1-5 | <5 |
| Example 1-6 | <5 |
| Example 1-7 | <5 |
| Comparative Example 1-3 | 36 |
| Comparative Example 1-4 | 39 |

From Table 2, it is seen that the deodorant processed cloths of Examples 1-5 to 1-7 had higher ketone-based gas adsorption effect and superior deodorizing performance than the deodorant processed cloths of Comparative Examples 1-3 and 1-4.

### 4. Production and Evaluation of Deodorant Resin Molded Articles

### Example 1-8

A mixture obtained by mixing 20 parts by mass of the gas adsorbent of Example 1-1 and 80 parts by mass of a polypropylene resin "PC630A" (trade name) manufactured by SunAllomer Ltd. was melt-kneaded at a temperature of 190°C using an extrusion molding machine to prepare a masterbatch. Subsequently, 10 parts by mass of this masterbatch and 90 parts by mass of the above-described propylene resin were injection-molded using an injection molding machine at a molding temperature of 190°C to obtain a plate-form test piece (100 mm × 100 mm × 2 mm).

Next, two of the thus obtained plate-form test pieces were placed in a 5-L test bag made of a vinyl alcohol-based polymer film, and the test bag was tightly sealed. Then, 3L of air containing 20 ppm of diacetyl was injected thereto. This test bag was left to stand at 20°C, and the diacetyl concentration in the test bag after 24 hours was measured using a gas detection tube. It is noted here that, when the diacetyl concentration in the test bag was measured by the same method without placing the plate-form test pieces therein and the thus measured value was taken as 100%, the rate of gas adsorption by the plate-form test pieces was determined to be 63%.

### Comparative Example 1-5

Plate-form test pieces were prepared and a gas adsorption test was performed in the same manner as in Example 1-8, except that ascorbic acid fatty acid ester powder was used in place of the gas adsorbent of Example 1-1. The gas adsorption rate was found to be 5%.

From the above, it is seen that the gas adsorbent of the invention has a ketone-based gas adsorption effect and exhibits excellent heat resistance also in molded articles processed at a high temperature.

### 5. Production and Evaluation of Gas Adsorbents (2)

### Example 2-1

An aqueous solution obtained by dissolving 1 g of white aminoguanidine hydrochloride in 10 mL of ion exchanged water was sprayed to 10 g of white amorphous aluminum silicate particles having a specific surface area of 610 m²/g and an average particle size of 6 µm, and the resultant was subsequently mixed at room temperature for 10 minutes.

It is noted here that the pH at which the amorphous aluminum silicate particles were dispersed in purified water at a concentration of 5% by mass was 6.5. Then, the thus obtained sprayed product was heated at 120°C for 50 hours, whereby about 11 g of white powder was obtained. Thereafter, the adsorption capacity of this adsorbent was measured for methyl ethyl ketone and diacetyl. The results thereof are shown in Table 3.

### Example 2-2

An aqueous solution obtained by dissolving 3 g of aminoguanidine hydrochloride in 10 mL of ion exchanged water was sprayed to 8 g of white MFI-type zeolite particles having a specific surface area of 350 m²/g and an average particle size of 6 µm, and the resultant was subsequently mixed at room temperature for 10 minutes. It is noted here that the pH at which the zeolite particles were dispersed in purified water at a concentration of 5% by mass was 6.6. Then, the thus obtained sprayed product was heated at 140°C for 35 hours, whereby about 11 g of white powder was obtained. Thereafter, the adsorption capacity of this adsorbent was measured for methyl ethyl ketone and diacetyl. The results thereof are shown in Table 3.

### Example 2-3

An aqueous solution obtained by dissolving 3 g of aminoguanidine hydrochloride in 10 mL of ion exchanged water was sprayed to 8 g of white silica gel particles having a specific surface area of 89 m²/g and an average particle size of 12 µm, and the resultant was subsequently mixed at room temperature for 10 minutes. It is noted here that the pH at which the silica gel particles were dispersed in purified water at a concentration of 5% by mass was 6.7. Then, the thus obtained sprayed product was heated at 130°C for 45 hours, whereby about 11 g of white powder was obtained. Thereafter, the adsorption capacity of this adsorbent was measured for methyl ethyl ketone and diacetyl. The results thereof are shown in Table 3.

### Example 2-4

The adsorption capacity of aminoguanidine hydrochloride powder was measured for methyl ethyl ketone and diacetyl. The results thereof are shown in Table 3.

### Comparative Example 2-1

An aqueous solution obtained by dissolving 3 g of ascorbic acid fatty acid ester in 10 mL of ion exchanged water was sprayed to 8 g of white silica gel particles having a specific surface area of 189 m²/g and an average particle size of 12 µm, and the resultant was subsequently mixed at room temperature for 10 minutes. It is noted here that the pH at which the silica gel particles were dispersed in purified water at a concentration of 5% by mass was 6.7. Then, the thus obtained sprayed product was heated at 140°C for 35 hours, whereby about 11 g of white powder was obtained. Thereafter, the adsorption capacity of this adsorbent was measured for methyl ethyl ketone and diacetyl. The results thereof are shown in Table 3.

### Comparative Example 2-2

The adsorption capacity of an activated charcoal "TAIKO CW350A" (trade name) manufactured by Futamura Chemical Co., Ltd. was measured for ketone-based gases (methyl ethyl ketone and diacetyl). The results thereof are shown in Table 3.

**(Table 3)**

| | Adsorption capacity (mL/g) | |
|---|---|---|
| | Methyl ethyl ketone | Diacetyl |
| Example 2-1 | 13 | 15 |
| Example 2-2 | 24 | 24 |
| Example 2-3 | 19 | 19 |
| Example 2-4 | 9 | 9 |
| Comparative Example 2-1 | 4 | 4 |
| Comparative Example 2-2 | 8 | 7 |

From Table 3, it is seen that the gas adsorbents of Examples 2-1 to 2-3 had greater ketone-based gas adsorption capacity and superior adsorption performance than the gas adsorbents of Comparative Examples 2-1 and 2-2.

### INDUSTRIAL APPLICABILITY

The ketone-based gas adsorbent of the invention has high adsorption performance for ketones such as acetone, methyl ethyl ketone, and diacetyl. This gas adsorbent is white in color and can thus be widely utilized. Further, when the gas adsorbent of the invention is in a powder form, it can be coated on or kneaded into products such as papers and fibers, whereby a variety of deodorant processed articles can be provided.

## Claims

1. A ketone-based gas adsorbent comprising a primary amine compound.

2. The ketone-based gas adsorbent according to claim 1, wherein the primary amine compound is at least one selected from the group consisting of hydrazide compounds, aminoguanidine compounds, and polyamines.

3. The ketone-based gas adsorbent according to claim 2, wherein the aminoguanidine compounds are aminoguanidine sulfate and aminoguanidine hydrochloride.

4. The ketone-based gas adsorbent according to any one of claims 1 to 3, which is a complex in which the primary amine compound is supported on an inorganic powder.

5. The ketone-based gas adsorbent according to claim 4, wherein the inorganic powder is at least one selected from silicate compounds, tetravalent metal phosphate compounds, silica gels, and zeolites.

6. The ketone-based gas adsorbent according to claim 4 or 5, wherein the inorganic powder has a BET specific surface area of not less than 80 m²/g.

7. The ketone-based gas adsorbent according to any one of claims 4 to 6, wherein the mass ratio of the primary amine compound and the inorganic powder is from 5:95 to 60:40.

8. A gas adsorbent composition comprising:
the ketone-based gas adsorbent according to any one of claims 1 to 7; and
at least one gas adsorbent selected from the group consisting of basic gas adsorbents, sulfur-based gas adsorbents, and organic acidic gas adsorbents.

9. A deodorant processed article comprising the ketone-based gas adsorbent according to any one of claims 1 to 7.
